# EUROPEAN PATENT APPLICATION

(11) **EP 1 634 578 A1**
(43) Date of publication of application: **15.03.2006**
(21) Application number: 05016011.8
(22) Date of filing: 22.07.2005
(51) Int. Cl.: A61K 8/81, A61Q 1/06, A61Q 1/10, A61Q 5/06, A61Q 17/04

(54) **Cosmetic compositions incorporating vinyl acetate-ethylene polymers**

(30) Priority: 27.07.2004 US 899586
(71) Applicant: AIR PRODUCTS POLYMERS, L.P., Allentown, PA 18195-1501 (US)
(72) Inventor: Hegedus, Charles Raymond, Allentown PA 18103 (US); Rabasco, John Joseph, Allentown PA 18104 (US); Thurau, Courtney T., Harleysville PA 19538 (US)
(74) Representative: Kador & Partner

(57) **Abstract**

This invention is directed to an improvement in cosmetic compositions comprised of a cosmetically acceptable carrier and a polymer. The improvement comprises a vinyl acetate-ethylene polymer formed by aqueous emulsion polymerization and having crystalline ethylene segments which imparts a thermal melt temperature, heat of fusion and tensile storage modulus to the polymer. The vinyl acetate-ethylene polymers have:
a crystalline melting point (Tₘ) ranging from 35 to 110 °C measured at a heat rate of 20 °C/minute and,
a tensile storage modulus of at least 1 x 10⁵ dynes/cm² at 115 °C and measured at 6.28 rad/sec.

## Description

### BACKGROUND OF THE INVENTION

Cosmetics and personal care products include a wide spectrum of consumer products, including hair care products (e.g., shampoos, conditioners, gels, mousse, hair styling aids, etc.), nail polish, lipstick, mascaras, makeup, eyeliners, eye shadows, moisturizing and sunscreen preparations. These products are produced in liquid, solid, and/or powder form depending on the application method and intended use. The term cosmetics will be used to refer to both cosmetic and personal care products.

Polymers and waxes tend to be primary and critical ingredients in many of these products since they greatly affect nearly all of their properties. Polymers carry many ingredients in the cosmetic formation, e.g., pigments and dyes, and provide mechanical strength, e.g., adhesion, cohesion, wear and water resistance, and they provide a physical barrier among other attributes. Other properties that polymers can provide to cosmetic products include rheology modification, hair setting and hold, and skin and hair conditioning. Typical polymers that are used in cosmetic formulas include vinyl acetate copolymers, polyacrylates, polymethacrylates, polyvinyl acetates, polystyrenes, and polyurethanes.

The following references are illustrative of the art with respect to cosmetic formulations:

US 6,534,071 discloses a cosmetic composition in the form of an oil-in water emulsion, generally free of surfactant and containing cellulose fibrils, which is suited for the treatment of the body's skin, face, hair, eyelashes, and lips. Oils suited for use in the cosmetic or dermatological compositions include avocado, apricot, mineral, and synthetic oils. Lipophilic gelling agents made of acrylic copolymers also are included.

US 6,248,336 discloses a cosmetic make-up composition suitable for use as a mascara and other eye make-ups which is in the form of an emulsion comprising an insoluble polymeric material and lipophilic oil components including a polyvinylpyrrolidone hexadecane copolymer. Insoluble polymeric materials are comprised of polymerized units of aromatics, vinyls, dienes, vinyl halides, alkyl esters of unsaturated carboxylic acids, hydroxy derivatives of alkyl esters of unsaturated carboxylic acids, amides, and the like. Waxes are also included in the cosmetic formulations include microcrystalline waxes, silicone waxes, beeswax and so forth.

US 6,534,047 discloses cosmetic compositions for coating keratin fibers such as eyelashes, hair, etc. comprising a cationic polymer, an anionic polymer and an aqueous dispersion of a C₁₋₆ alkyl (meth)acrylate. Examples of alkyl acrylates include copolymers of ethyl and methyl acrylate, methyl methacrylate and butyl acrylate. Examples of anionic polymers include copolymers of monomers having carboxylic, sulfonic and phosphoric acid. Cationic polymers are those polymers such as polyamines, polyaminoamides and polyquartemary ammoniums.

US 6,509,009 discloses a lipstick comprising between about 0.1 and about 20 parts of an alcohol soluble and water insoluble resin such as polyvinyl acetate, between about 0.1 and about 15 parts of a cellulose, between about 0.1 and about 15 parts of a cosmetic pigment, and between about 50 and 99 parts of an organic solvent.

US 5,041,281 discloses an oil in water emulsion sunscreen composition including from 0.5 and about 20 weight percent of a copolymer of ethylene and vinyl acetate and from 0.5 and about 10 weight percent of a second film forming polymer which increases the substantivity of the composition. The sunscreen composition also includes between about 0.5 and about 10 percent of an emulsifier and between about 1 and about 30 percent of an oil soluble sunscreen agent.

US 4,935,228 discloses a lip gloss comprising a mineral oil gel comprising polyethylene and an ethylene-vinyl acetate copolymer, wherein the weight proportion of polyethylene to ethylene-vinyl acetate copolymer is in the range of 90:10 to 70:30, respectively where polyethylene is present in major proportion, and wherein the weight proportion of polyethylene to ethylene-vinyl acetate copolymer is in the range of 20:80 to 30:70.

US 5,505,935 discloses a cosmetic sunscreen composition that includes an ethylene/vinyl acetate copolymer, an acrylic polymer such as poly(methyl methacrylate) and a chromophoric organic sunscreen agent capable of absorbing ultraviolet radiation within the range 290 to 400 nm. The ethylene/vinyl acetate copolymer and acrylic polymer have been found to interactively boost the SPF value of the organic sunscreen.

US 6,517,823 discloses a high gloss mascara comprised of a glossy film former, thickener and wax. Examples of glossy film formers include vinyl acetate/acrylates, styrene/acrylates, vinyl acetate-ethylene copolymers polyvinyl alcohol and the like.

US 6,497,864 discloses a hairstyling composition in a cosmetically acceptable medium comprises a non-ionic film forming polymer. Examples of film forming polymers include vinyl acetate/acrylic esters, vinyl acetate-ethylene, vinyl acetate/maleic acid esters, alkyl acrylate polymers and the like.

### BRIEF SUMMARY OF THE INVENTION

This invention is directed to an improvement in cosmetic compositions comprised of a cosmetically acceptable carrier and a polymer. The improvement comprises a vinyl acetate-ethylene polymer formed by aqueous emulsion polymerization and having crystalline ethylene segments which imparts a thermal melt temperature, heat of fusion and tensile storage modulus to the polymer. The vinyl acetate-ethylene polymers have:
a crystalline melting point (Tₘ) ranging from 35 to 110 °C and preferably from 50 to 90 °C; measured at a heat rate of 20 °C/minute and,
a tensile storage modulus of at least 1 x 10⁵ dynes/cm² at 115 °C and measured at 6.28 rad/sec.

Significant advantages can be achieved in cosmetic formulations employing the aqueous emulsion polymerized vinyl acetate-ethylene polymer having crystalline ethylene segments and these include:
an ability to formulate cosmetic compositions incorporating a vinyl acetate-ethylene polymer having a wax-like feel;
an ability to produce cosmetic formulation that have excellent thermal properties for heat setting;
an ability to formulate cosmetic compositions having good adhesive characteristics, and,
an ability to formulate cosmetic compositions having good adhesion to keratinous fibers required in mascaras and hair styling sprays.

### DETAILED DESCRIPTION OF THE INVENTION

As described in the background of the invention, cosmetic and personal care compositions have wide applications ranging from use on keratin substrates, e.g., hair styling, eye enhancing mascaras, to makeups, sunscreens and the like. They are formulated in liquid (e.g., sprayable forms) and soft or solid emulsions. Many of the cosmetic formulations are oil-in-water emulsions. Common to most of the cosmetic compositions, there are the requirements of adhesion to the skin and hair, non smearing and water resistance.

Cosmetically acceptable or pharmacological carriers employed in cosmetic compositions often are comprised of water, and one or more oils, waxes, emollients surfactants, alcohols, organic solvents, pigments, plasticizers, vitamins, thickeners, sunscreen agents, antioxidants, and gloss enhancers. Included in the compositions are polymers. The concentration of each of the respective ingredients in the cosmetic formulation is varied depending upon the application and desired effect of the cosmetic.

The main properties of the aqueous emulsion polymerized vinyl acetate-ethylene polymers containing crystalline ethylene segments that make them suitable for cosmetic applications are: waterborne (no organic solvents), hydrophobic, oleophobic, low surface energy, high and low gloss films, potential for heat activation, and the film formation properties can be varied depending upon the required performance attributes of the finished cosmetic formulation. These properties are a direct result of the polymer composition and morphological structure associated with the amorphous and crystalline ethylene segments in the copolymers.

In conventional vinyl acetate-ethylene polymers formed by aqueous emulsion polymerization, the ethylene units are largely incorporated in an amorphous state and there is a substantial absence of crystalline ethylene domains. The absence of crystalline ethylene segments diminishes many desirable attributes compared to vinyl acetate-ethylene polymers having crystalline ethylene segments.

In the improved cosmetic compositions described herein, a polymer is included in combination with the cosmetically acceptable carrier, and the polymer is one that is based upon a polymer of vinyl acetate and ethylene. The significant difference between the aqueous emulsion polymerized vinyl acetate-ethylene polymers for use with the cosmetic formulation described herein and the vinyl acetate-ethylene polymers employed heretofore is in the presence of crystalline ethylene segments which impart a thermal melt temperature, heat of fusion and tensile storage modulus to the polymer. The crystalline melting point (Tₘ) of the vinyl acetate-ethylene polymers ranges from 35 to 110 °C and preferably from 50 to 90 °C; measured via differential scanning calorimetry at a heat rate of 20 °C/minute. The tensile storage modulus ranges from of at least 1 x 10⁵ dynes/cm² at 115 °C measured at 6.28 rad/sec and generally greater than 2 X10⁵ dynes/cm². In addition, the polymers have a crystalline heat of fusion (H_{f}) ranging from 5 to 100 joules per gram (J/g), and preferably 10 to 70 J/g. The glass transition temperature (T_{g}) of the polymers ranges from + 25 °C to about -35 °C

The aqueous emulsion polymerized vinyl acetate-ethylene polymers for use in the cosmetic formulations are based upon vinyl acetate and ethylene with the level of polymerized units of vinyl acetate ranging from 15 to 90% by weight of the polymer and the level of polymerized units of ethylene ranging from 10% to 85% by weight; preferably from 25 to 80 weight percent vinyl acetate and 20 to 75% by weight ethylene; and most preferably from 35 to 75% by weight vinyl acetate and 25 to 65% by weight ethylene.

Other monomers which can be emulsion polymerized into the polymer include, but are not limited to C₁-C₁₈ alkyl esters or esters with C₁-C₁₈ alkanols, such as methanol, ethanol, propanol, butanol, and 2-ethylhexanol, C₁ to C₁₅ alkyl vinyl ester, a C₁ to C₁₅ alkyl acrylate or a C₁ to C₁₅ alkyl methacrylate, such as methyl (meth)acrylate, ethyl (meth)acrylate, propyl (meth)acrylate, butyl (meth)acrylate, and 2-ethylhexyl (meth)acrylate, a C₁ to C₆ hydroxyalkyl (meth)acrylate, such as, hydroxyethyl (meth)acrylate and hydroxypropyl (meth)acrylate, a C₁ to C₁₅ alkyl maleate, C₁ to C₁₅ alkyl fumarate, C₃-C₁₀ alkenoic acids, such as acrylic acid, methacrylic acid, crotonic acid, and isocrotonic acid, methacrylic acid, vinyl halides, such as vinyl chloride; mono and diesters of alpha, beta-unsaturated C₄-C₁₀ alkenedioic acids such as maleic acid, fumaric acid, and itaconic acid and C₁-C₁₈ alkanols; nitrogen containing mono-olefinically unsaturated monomers, particularly nitriles, amides, N-methylol amides, C₁-C₄ alkanoic acid ethers of N-methylol amides and allylcarbamates, such as acrylonitrile, acrylamide, methacrylamide, N-methylol acrylamide, N-methylol methacrylamide, N-methylol allylcarbamate, and C₁-C₄ alkyl ethers or C₁-C₄ alkanoic acid esters of N-methylol acrylamide, N-methylol methacrylamide and N-methylol allylcarbamate; sodium vinyl sulfonate; and 2-acrylamido-2-methyl propanesulfonate. The monomers can be incorporated in minor amounts, e.g. from 0 to about 10% by weight.

Imparting cationic character to the polymer is also advantageous for many cosmetic applications. This can be accomplished for the polymers of this invention by copolymerizing minor amounts (typically less than 10 wt%) of amine functional monomers and/or utilizing cationic surfactants during the emulsion polymerization. Representative amine functional monomers which can be emulsion polymerized into the polymer include monoalkylaminoalkyl methacrylate, monoalkylaminoalkyl acrylate, dialkylaminoalkyl methacrylate, trialkylammoniumalkyl methacrylate, trialkylammoniumalkyl acrylate, or dialkylaminoalkyl acrylate, or dialkylaminoalkyl (meth)acrylamides and the like. The alkyl groups of the above cited functional monomers can be substituted or unsubstituted C₁ to C₆ alkyl groups. Examples of appropriate monomers are 2-(dimethylamino)ethyl methacrylate, 2-(diethylamino)ethyl methacrylate, 2-(dimethylamino)ethyl acrylate, 3-(dimethylamino)-2,2-dimethylpropyl acrylate, 2-(diethylamino)ethyl acrylate, 2-(*tertiary*-butylamino)ethyl methacrylate, 2-(trimethylammonium)ethyl methacrylate chloride, 2-(trimethylammonium)ethyl acrylate chloride, 3-(dimethylamino)propyl methacrylamide, methacrylamidopropyl trimethylammonium chloride, diallyldimethylammonium chloride, vinylpyridine, allylamine, or combinations thereof.

One preferred way to enhance crystalline domain formation of ethylene in the vinyl acetate-ethylene polymer is to delay the addition of vinyl acetate during the polymerization process such that the unreacted vinyl acetate level present in the reactor is minimal at different stages during the process, i.e., below 5% unreacted free vinyl acetate monomer. In one preferred mode, it is preferred to stage the addition of vinyl acetate in the polymerization process over an initial period of time. Typically, one completes the addition within 75% of the total polymerization period. Thus, vinyl acetate-ethylene polymerization can take place in the one stage where most, but not all, of the ethylene will reside in amorphous regions, and the formation of the majority of crystalline ethylene domains can occur in another stage of the polymerization process.

In the preferred process for effecting aqueous emulsion polymerization and the formation of vinyl acetate-ethylene polymers for cosmetic applications, polymerization of ethylene, vinyl acetate, and optionally other comonomers is initiated by thermal initiators or by redox systems. A thermal initiator is typically used at temperatures of about 60 °C or higher, preferably about 70 °C or higher. Redox systems can be used over a wide range of temperatures, but are typically used at temperatures of about 60 °C or lower. The amount of initiator used in the process typically is substantially higher than used in prior processes for forming standard aqueous based vinyl acetate-ethylene emulsion polymers. Typically, the level of initiator is at least 0.5% and typically greater than 0.7% by weight of the total monomer charged. In addition, it is preferred that the initiator is added over the time of polymerization. It is believed that a high radical flux created by the higher levels of initiator facilitates ethylene incorporation during this low pressure polymerization process and leads to crystalline ethylene segments and a branched polymer architecture in the resulting polymer and thus exhibits a higher tensile storage modulus at elevated temperatures, thermal melting point, and a heat of fusion. Thermal initiators are well known in the emulsion polymer art and include, for example, ammonium persulfate, sodium persulfate, and the like. Suitable redox systems are based upon reducing agents and peroxides. Combinations of reducing agents, such as sodium formaldehyde sulfoxylate or erythorbates and peroxides such as t-butyl hydroperoxide (t-BHP) and hydrogen peroxide are representative. Erythorbates and other formaldehyde-free reducing agents are preferred.

The tensile storage modulus profile for these vinyl acetate-ethylene polymers having crystalline ethylene segments provides an insight to the distribution of vinyl acetate and ethylene in the polymer and the melt flow characteristics. The polymers have a high tensile storage modulus and are highly viscous with minimal flow properties at temperatures where other vinyl acetate-ethylene and VAE polymers melt and exhibit melt flow characteristics. The polymers described herein maintain a high viscosity and resistance to flow at temperatures well above their melt temperatures. The modulus should be at least 1 x 10⁵ in dynes/cm², (preferably 2 x 10⁵) at 115 °C as measured at a test frequency of 6.28 rad/sec.

Other factors leading to crystalline ethylene domains within the polymer is the pressure and temperature of polymerization. Although pressure is influential in achieving higher ethylene concentration levels in the polymer, it also is a factor in determining whether the amount of ethylene which is present is in amorphous regions or crystalline domains. Temperature, also is relevant in the formation of ethylene crystallinity. Lastly, the level of initiator is also a factor in developing vinyl acetate-ethylene polymers for cosmetic applications.

Chain transfer agents, water soluble or oil soluble, can be use during the polymerization and formation of vinyl acetate-ethylene polymers for cosmetic applications. Any of the common chain transfer agents known in the emulsion polymerization art can be used, such as mercaptan derivatives. Dodecylmercaptan is an example of an oil soluble chain transfer agent. For example, dodecylmercaptan can be dissolved in vinyl acetate monomer and introduced to the reactor via the monomer delay feed. Chain transfer agents are typically used in amounts less than 2.0 weight percent, based on total polymer weight.

The stabilizing system for aqueous emulsion polymerization can comprise surfactants and/or emulsifiers. It can also contain polymeric colloids, such as polyvinyl alcohol and cellulosic colloids, such as hydroxyethyl cellulose (HEC), optionally in combination with surfactants or emulsifiers. The colloids can be used in amounts of about 0.1 to 10 wt%, preferably 0.5 to 5 wt%, based on the total monomers.

The surfactant or emulsifier can be used at a level of about 1 to 10 wt%, preferably 1.5 to 6 wt%, based on the total weight of monomers and can include any of the known and conventional surfactants and emulsifying agents, principally the nonionic, anionic, and cationic materials, heretofore employed in emulsion polymerization. Among the anionic surfactants found to provide good results are alkyl sulfates and ether sulfates, such as sodium lauryl sulfate, sodium octyl sulfate, sodium tridecyl sulfate, and sodium isodecyl sulfate, sulfonates, such as dodecylbenzene sulfonate, alpha olefin sulfonates and sulfosuccinates, and phosphate esters, such as the various linear alcohol phosphate esters, branched alcohol phosphate esters, and alkylphenolphosphate esters.

Examples of suitable nonionic surfactants include the Igepal surfactants which are members of a series of alkylphenoxy-poly(ethyleneoxy)ethanols having alkyl groups containing from about 7 to 18 carbon atoms, and having from about 4 to 100 ethyleneoxy units, such as the octylphenoxy poly(ethyleneoxy)ethanols, nonylphenoxy poly(ethyleneoxy)ethanols, and dodecylphenoxy poly(ethyleneoxy)ethanols. Others include fatty acid amides, fatty acid esters, glycerol esters, and their ethoxylates, ethylene oxide/propylene oxide block polymers, secondary alcohol ethoxylates, and tridecylalcohol ethoxylates.

Some of the essential properties of cosmetic formulations for hair sprays, lip gloss, mascara, makeup and the like which are provided by their materials in the formulations, are:

| Spreadability - the ability to be spread onto a specific application area to a consistent desired thickness while feeling comfortable | Film characteristics |
|---|---|
| Attractive feel - soft and smooth | Moisturizing |
| Water resistance | Flexibility |
| Abrasion resistance | Toughness |
| Gloss (high or low) | Low toxicity and odor |
| Ease of removal | |

A wide variety of cosmetic formulations can be prepared using the vinyl acetate-ethylene polymers having crystalline ethylene segments. Oil-in water emulsions have wide application for use in cosmetic applications and such formulations can be prepared using the aqueous emulsion polymerized vinyl acetate-ethylene polymers as a component. The oily phase may contain any of the fatty substances, and especially oils, conventionally used in the cosmetic or dermatological fields. Among the oils that can be used in the vinyl acetate-ethylene emulsion may be made, for example, of plant oils such as jojoba, avocado, soft-almond, apricot and corn oils and the liquid fraction of karite butter; mineral oils such as liquid paraffin and hydrogenated polyisobutylene; synthetic oils such as 2-ethylhexyl palmitate, isopropyl myristate, hydrogenated isoparaffin, isononyl isononanoate and cetearyl octanoate; volatile or non-volatile silicone oils and fluorinated oils.

Waxes have been widely used in formulating cosmetic products. They provide soft feel, low surface energy, water repellency (hydrophobicity), and spreadability (ease of application). Waxes can be chosen from known animal, fossil, vegetable, mineral or synthetic waxes, such as paraffin waxes, polyethylene waxes, carnauba or candelilla waxes, beeswaxes, lanolin wax, Chinese insect waxes, rice wax, ouricury wax, esparto wax, cork fibre wax, sugarcane wax, japan wax, sumach wax, montan wax, microcrystalline waxes, ozokerite, the waxes obtained by the Fischer-Tropsch synthesis, silicone waxes or their mixtures. However, because the vinyl acetate-ethylene polymers of this invention have crystalline ethylene segments and the waxy feel, it is possible to reduce and eliminate the need for waxes as this characteristic is supplied by the polymer. Similarly, it is possible to reduce and/or eliminate the need for oils and organic esters as the wax component of these polymers supply substantially similar benefits.

The following examples are provided to illustrate various embodiments of the invention and are not intended to restrict the scope thereof. Ethylene levels in the polymers were determined by mass balance.

### TENSILE STORAGE MODULUS

Tensile storage modulus as a function of temperature was measured at a test frequency of 6.28 rad/sec and expressed as dynes/cm². More specifically, dynamic mechanical testing of the polymer samples for measuring tensile storage modulus was accomplished using the following procedure. ASTM-D-4065-94 and ASTM-D-5026-94 were used as guidelines for this procedure. Each polymer emulsion was cast as a film and allowed to dry a minimum of several days at ambient conditions. The dry film thickness was typically in the range of 0.3 to 0.5 mm. For samples that did not film form adequately at room temperature, the polymers were compression molded at 100 to 150 °C. The specimens used for testing were die cut from the film and were about 6.3 mm wide and 30 mm long. The specimens were tested on a Rheometrics Solid Analyzer (RSA II), from Rheometric Scientific, Inc., to obtain the tensile dynamic mechanical properties. Data were obtained every 6 °C over the -100 to 200 °C range using a fiber/film fixture and a deformation frequency of 6.28 rad/sec. To help ensure linear viscoelastic conditions, the applied strains were typically 0.05% in the glassy region and up to 1% in the rubbery region. A soak time of one minute was used at each temperature to ensure isothermal conditions. For each temperature, the RSA II calculated the tensile storage modulus (E'), tensile loss modulus (E"), and tangent delta (tan δ) based on the width, thickness and length of the sample.

### MEASUREMENT OF T_{g}, Tₘ, AND H_{f}

T_{g}, Tₘ, and H_{f} were determined via differential scanning calorimetry (DSC) using a TA Instruments Thermal Analyst 3100 with DSC 2010 module. Polymer samples were thoroughly dried prior to testing. Samples were held at 100 °C in the calorimeter for 5 minutes, cooled to -75 °C, and then the scan acquired at a heating rate of 20 °C per minute up to a final temperature of 200 °C. The T_{g} corresponds to the extrapolated onset values obtained from the baseline shift at the glass transition during the heating scan. The melting point temperature corresponds to the peak in the heat flow curve. The heat of fusion was calculated by integrating the area under the melting endotherm; the baseline for this integration was constructed by extrapolating the linear region of the heat flow curve after the melt, back to the point of intersection with the heat flow curve before the melt.

### Example 1

A polymer emulsion containing crystalline ethylene segments was prepared by first charging a one-gallon stainless steel pressure reactor with the following mixture:

| Material | Mass charged, g |
|---|---|
| DI Water | 700 |
| Rhodacal DS-10 | 9 |
| Natrosol 250GR (2% aqueous solution) | 500 |
| Ferrous ammonium sulfate (2% aqueous solution) | 6 |
| Monomer Solution comprising 94.71 wt% vinyl acetate, 4.71 wt% acrylic acid, and 0.571 wt% dodecylmercaptan | 120 |

The following delay mixtures were utilized:

| Material | Mass charged, g |
|---|---|
| Aqueous 4.0 % t-BHP | 130 |
| Aqueous 8% sodium formaldehyde sulfoxylates | 139 |
| Aqueous solution containing 52.5 g Rhodacal DS-10 and 297.5 g water | 306 |
| Monomer Solution comprising 94.71 wt% vinyl acetate, 4.71 wt% acrylic acid, and 0.571 wt% dodecylmercaptan | 540 |
| Ethylene | 1400 psig for 5.5 hours |

Agitation at 100 rpm was begun with a nitrogen purge. Agitation was then increased to 900 rpm and the reactor heated to 60 °C. After pressurizing the reactor with ethylene to 1400 psig, 10 g of 8% aqueous sodium formaldehyde sulfoxylate was added to the reactor. Delay feeds of tert-butylhydrogen peroxide (4%) at 0.4 g/min. and 8% sodium formaldehyde sulfoxylate at 0.4 g/min were begun. After a 10 minute period, the surfactant delay was begun at 0.85 g/min and the monomer solution delay was begun at 3.0 g/min. Redox rates were adjusted during the reaction period to maintain reasonable reaction rates. Ethylene pressure of 1400 psig was maintained for 5.5 hours. The monomer solution delay was turned off at the 3 hour mark. The ethylene makeup valve was closed at the 5.5 hour mark. The surfactant delay and initiator delay were stopped at the 6 hour mark. The reaction was then cooled to 35 °C, transferred to a degasser to remove unreacted ethylene, and 2 g of Rhodaline 675 defoamer was added. The following properties of the resulting emulsion polymer were measured:

| Polymer Composition (By mass balance calculation) | 42.5% Ethylene 55% Vinyl acetate 2.5% Acrylic Acid |
|---|---|
| T_{g} Onset (°C) | -27.1 |
| Viscosity (60 rpm) (cps) | 348 |
| % solids | 41.9 |
| pH | 4.0 |
| Tₘ (°C) / Heat of Fusion (J/g) | 89.9 / 15.8 |

### Example 2

A polymer emulsion containing crystalline ethylene segments was prepared by the following procedure: A one-gallon stainless steel pressure reactor was charged with the following mixture:

| Material | Mass charged, g |
|---|---|
| DI Water | 1100 |
| Aerosol MA801 | 10 |
| Monomer solution consisting of 94.71 wt% vinyl acetate, 4.71 wt% acrylic acid, and 0.571 wt% dodecylmercaptan | 120 |

| | |
|---|---|
| Aerosol MA80I supplied by Cytec | |

The following delay mixtures were utilized:

| Material | Mass charged, g |
|---|---|
| Aqueous 10.0 wt% ammonium persulfate containing 4.0 wt% sodium bicarbonate | 127 |
| Rhodacal DS-10, diluted to 15% active | 260 |
| Monomer solution consisting of 94.71 % vinyl acetate, 4.71 % acrylic acid, and 0.571 % dodecylmercaptan | 540 |
| Ethylene | 1400 psig for 5.5 hours |

| | |
|---|---|
| Rhodacal DS-10 supplied by Rhodia | |

Agitation at 100 rpm was begun with a nitrogen purge. Agitation was then increased to 900 rpm and the reactor heated to 80 °C. After pressurizing the reactor with ethylene to 1400 psig, 15 g of initiator solution was added at a rate of 5.0 grams/minute. When the 15 grams of initiator had been added, the initiator delay rate was reduced to 0.30 grams/minute. At the 15 minute mark, the monomer delay was begun at 3.0 g/minute and the surfactant delay was begun at 0.72 g/minute. Ethylene pressure of 1400 psig was maintained for 5.5 hours. The vinyl acetate delay was stopped at the 3 hour mark. The ethylene supply was stopped at the 5.5 hour mark. The surfactant delay and the initiator delay were stopped at the 6 hour mark, followed by holding the reaction mixture at temperature for another 30 minutes. The reaction was then cooled to 40 °C, transferred to a degasser, and 2 g of Rhodaline 675 defoamer was added. The following properties of the resulting emulsion polymer were measured:

| Polymer Composition (by mass balance calculation) | 49% Ethylene 48.6% Vinyl acetate 2.4% Acrylic acid |
|---|---|
| T_{g} Midpoint (°C) | -27.3 |
| Viscosity (60 rpm) (cps) | 880 |
| % solids | 48.3 |
| pH | 4.66 |
| Tₘ (°C) / Heat of Fusion (J/g) | 90.2/19.6 |

### Example 3

A polymer emulsion containing crystalline ethylene segments was prepared by the following procedure: A one-gallon stainless steel pressure reactor was charged with the following mixture:

| Material | Mass charged, g |
|---|---|
| DI Water | 1100 |
| Aerosol MA80I | 10 |
| Monomer solution consisting of 95 wt% vinyl acetate and 5 wt% acrylic acid | 120 |

| | |
|---|---|
| Aerosol MA80I supplied by Cytec | |

The following delay mixtures were utilized:

| Material | Mass charged, g |
|---|---|
| Aqueous 10.0 wt% ammonium persulfate containing 4.0 wt% sodium bicarbonate | 131 |
| Rhodacal DS-10, diluted to 15% active | 260 |
| Monomer solution consisting of 95 wt% vinyl acetate and 5 wt% acrylic acid | 540 |
| Ethylene | 1400 psig for 5.5 hours |

| | |
|---|---|
| Rhodacal DS-10 supplied by Rhodia | |

Agitation at 100 rpm was begun with a nitrogen purge. Agitation was then increased to 900 rpm and the reactor heated to 80 °C. After pressurizing the reactor with ethylene to 1400 psig, 15 g of initiator solution was added at a rate of 5.0 g/minute. When the 15 grams of initiator had been added, the initiator delay rate was reduced to 0.30 grams/minute. At the 15 minute mark, the monomer delay was begun at 3.0 g/minute and the surfactant delay was begun at 0.72 g/minute. Ethylene pressure of 1400 psig was maintained for 5.5 hours. The vinyl acetate delay was stopped at the 3 hour mark. The ethylene supply was stopped at the 5.5 hour mark. The surfactant delay and the initiator delay were stopped at the 6 hour mark, followed by holding the reaction mixture at temperature for another 30 minutes. The reaction was then cooled to 40 °C, transferred to a degasser, and 2 g of Rhodaline 675 defoamer was added. The following properties of the resulting emulsion polymer were measured:

| Polymer Composition (by mass balance calculation) | 51 % Ethylene 46.5% Vinyl acetate 2.5% Acrylic acid |
|---|---|
| Tg Midpoint (°C) | -29.9 |
| Viscosity (60 rpm) (cps) | 400 |
| % solids | 49.1 |
| pH | 4.53 |
| Tm (°C) / Heat of Fusion (J/g) | 86.6 / 23.6 |

### Example 4

The procedure of Example 3 was followed, except 700 psig ethylene pressure was maintained for the first 3 hours of the polymerization followed by 1400 psig ethylene pressure for the next 2.5 hours of the polymerization. The following properties of the resulting emulsion polymer were measured:

| Polymer Composition (by mass balance calculation) | 42% Ethylene 55.1% Vinyl acetate 2.9% acrylic acid |
|---|---|
| Tg Onset (°C) | - 6.7 |
| Viscosity (60 rpm) (cps) | 1100 |
| % solids | 45.5 |
| pH | 4.53 |
| Tₘ (°C) / Heat of Fusion (J/g) | 91.3 / 24.2 |

### Example 5

A three-gallon stainless steel pressure reactor was charged with the following mixture:

| Material | Mass charged, g |
|---|---|
| DI Water | 3300 |
| Aerosol MA-80I | 30 |
| Vinyl Acetate | 360 |

The following delay mixtures were utilized:

| Material | Mass charged, g |
|---|---|
| Aqueous 10.0 % ammonium persulfate containing 4.0 % sodium bicarbonate | 345 |
| Aqueous 15.0 % diluted solution of Rhodacal DS-10 | 795 |
| Vinyl Acetate | 1655 |
| N-methylolacrylamide (48%) (NMA) | 419 |
| Ethylene | 1400 psig for 5.5 hours |

Agitation at 100 rpm was begun with a nitrogen purge. Agitation was then increased to 600 rpm and the reactor heated to 80 °C. After pressurizing the reactor with ethylene to 1400 psig, 15 g of initiator solution was added at a rate of 5.0 g/min. After the 15 grams of initiator were in the reactor, the initiator delay rate was reduced to 0.90 g/min. At initiation, the vinyl acetate delay was begun at 9.0 g/min, the surfactant delay was begun at 2.16 g/min, and the NMA delay was begun at 1.17 g/min. Ethylene pressure of 1400 psig was maintained for 5.5 hours. The vinyl acetate delay was stopped at the 3 hour mark. The ethylene supply was stopped at the 5.5 hour mark. The surfactant delay, NMA delay, and initiator delay were stopped at the 6 hour mark, followed by holding the reaction mixture at temperature for another 30 minutes. The reaction was then cooled, transferred to a degasser, and 2 g of Rhodaline 675 defoamer was added. The following properties of the resulting emulsion polymer were measured:

| Polymer Composition (by mass balance calculation) | 43% Ethylene 52% Vinyl acetate 5% NMA |
|---|---|
| T_{g} Onset (°C) | -29.6 |
| Viscosity (60 rpm) (cps) | 53 |
| % solids | 47.2 |
| pH | 5.2 |
| Tₘ (°C) / Heat of Fusion (J/g) | 79.0 / 12.2 |

### Example 6

A polymer emulsion containing crystalline ethylene segments was prepared by first charging a one-gallon stainless steel pressure reactor with the following mixture:

| Material | Mass charged, g |
|---|---|
| DI Water | 700 |
| Rhodacal DS-10 | 9 |
| Natrosol 250GR (2% aqueous solution) | 500 |
| Ferrous ammonium sulfate (2% aqueous solution) | 6 |
| Monomer Solution comprising 94.71 wt% vinyl acetate, 4.71 wt% acrylic acid, and 0.571 wt% dodecylmercaptan | 150 |

The following delay mixtures were utilized:

| Material | Mass charged, g |
|---|---|
| Aqueous 4.0 % t-BHP | 118 |
| Aqueous 8% sodium formaldehyde sulfoxylates | 127 |
| Aqueous solution containing 52.5 g Rhodacal DS-10 and 297.5 g water | 253 |
| Monomer Solution comprising 94.71 wt% vinyl acetate, 4.71 wt% acrylic acid, and 0.571 wt% dodecylmercaptan | 540 |
| Ethylene | 700 psig for 3 hours; 1400 psig for 3 hours |

Agitation at 100 rpm was begun with a nitrogen purge. Agitation was then increased to 900 rpm and the reactor heated to 60 °C. After pressurizing the reactor with ethylene to 700 psig, 10 g of 8% aqueous sodium formaldehyde sulfoxylate was added to the reactor. Delay feeds of tert-butylhydrogen peroxide (4%) at 0.3 g/minute and 8% sodium formaldehyde sulfoxylate at 0.3 g/minute were begun. At initiation, the surfactant delay was begun at 0.65 g/min and the monomer solution delay was begun at 3.0 g/min. Ethylene pressure of 700 psig was maintained for 3 hours. At the 3 hour mark, the monomer solution delay was turned off and the ethylene pressure was increased to 1400 psig. At the 6 hour mark, the ethylene makeup valve was closed. The surfactant delay and redox delays were stopped at the 6.5 hour mark. The reaction was then cooled to 40 °C, transferred to a degasser to remove unreacted ethylene, and 2 g of Rhodaline 675 defoamer was added. The following properties of the resulting emulsion polymer were measured:

| Polymer Composition (by mass balance calculation) | 39% Ethylene 58% Vinyl acetate 3% Acrylic Acid |
|---|---|
| T_{g} Onset (°C) | - 9.7 |
| Viscosity (60 rpm) (cps) | 920 |
| % solids | 42.0 |
| pH | 4.27 |
| Tₘ (°C) / Heat of Fusion (J/g) | 95.7 / 28.2 |

### Example 7

A polymer emulsion containing crystalline ethylene segments was prepared by first charging a one-gallon stainless steel pressure reactor with the following mixture:

| Material | Mass charged, g |
|---|---|
| DI Water | 1000 |
| Rodapon UB sodium lauryl sulfate | 42 |
| Celvol 205 poly(vinyl alcohol) (10% water solution) | 200 |
| Vinyl Acetate | 21 |

| | |
|---|---|
| Celvol 205 : 86-88 % hydrolyzed poly(vinyl alcohol) supplied by Celanese | |

The following delay mixtures were utilized:

| Material | Mass charged, g |
|---|---|
| Aqueous 10.0 % ammonium persulfate containing 3.5% sodium bicarbonate | 115 |
| Aqueous 10.0% sodium lauryl sulfate | 218 |
| Vinyl Acetate | 325 |
| Ethylene | 1800 psig for 5 hours |

Agitation at 100 rpm was begun with a nitrogen purge. Agitation was then increased to 800 rpm and the reactor heated to 80 °C. After pressurizing the reactor with ethylene to 1800 psig (12,512 kPa), 5 g of initiator was added at a rate of 5.0 g/min. At initiation, the vinyl acetate delay was begun at 1.08 g/min, the surfactant delay was begun at 0.71 g/min, and the initiator delay was re-started at 0.34 g/min. Ethylene pressure of 1800 psig (12,512 kPa) was maintained for 300 minutes. The vinyl acetate delay, surfactant delay, initiator delay, and ethylene pressure were completed at the 300 minute mark. Then the reaction mixture was held at temperature for another 30 minutes. Next, the reaction was cooled to 30 °C and transferred to a degasser and 2 g of Rhodaline 675 defoamer was added. The following properties of the resulting emulsion polymer were measured:

| Polymer Composition (by mass balance calculation) | 70% Ethylene 30% Vinyl acetate |
|---|---|
| T_{g} Onset (°C) | -40.15 |
| Viscosity (60 rpm) (cps) | 4700 |
| % solids | 45.9 |
| pH | 4.90 |
| Tm (°C) / H_{f} (J/g) | 69.2/42 |

### Example 8

A polymer emulsion containing crystalline ethylene segments was prepared by first charging a one-gallon stainless steel pressure reactor with the following mixture:

| Material | Mass charged, g |
|---|---|
| DI Water | 1000 |
| Rhodapon UB sodium lauryl sulfate | 60 |
| Natrosol 250GR HEC | 500 |
| Vinyl Acetate | 150 |

The following delay mixtures were utilized:

| Material | Mass charged, g |
|---|---|
| Aqueous 10.0 % Ammonium persulfate containing 3.5 % sodium bicarbonate | 63 |
| Aqueous 10.0 % Sodium lauryl sulfate | 221 |
| Ethylene | 1800 psig for 3 hours |

Agitation at 100 rpm was begun with a nitrogen purge. Agitation was then increased to 800 rpm and the reactor heated to 80 °C. After pressurizing the reactor with ethylene to 1800 psig (12,512 kPa), 5 g of initiator was added at a rate of 5.0 g/min. After 5 g of initiator had been added, the initiator delay feed rate was reduced to 0.30 g/min. After a 10 minute period, the surfactant delay was begun at 1.22 g/min. Ethylene pressure of 1800 psig was maintained for 180 minutes. The surfactant delay, initiator delay, and ethylene pressure were turned off at the 180 minute mark, followed by holding the reaction mixture at temperature for another 30 minutes. The reaction was then cooled to 30 °C, transferred to a degasser, and 2 g of Rhodaline 675 defoamer was added. The following properties of the resulting emulsion polymer were measured:

| Polymer Composition (by mass balance calculation) . | 83% Ethylene 17% Vinyl acetate |
|---|---|
| T_{g} Onset (°C) | -35.6 |
| Viscosity (60 rpm) (cps) | 30 |
| % solids | 34.5 |
| pH | 5.6 |
| Tm (°C) / H_{f} (J/g) | 96.7/72.5 |

### Example 9

A one-gallon stainless steel pressure reactor was charged with the following mixture:

| Material | Mass charged, g |
|---|---|
| DI Water | 700 |
| Rhodacal DS-10 | 17 |
| Natrosol 250GR (2% aqueous solution) | 500 |
| Ferrous ammonium sulfate (2% aqueous solution) | 6 |
| Monomer Solution comprising 94.76 wt% vinyl acetate, 4.71 wt% acrylic acid, and 0.529 wt% dodecylmercaptan | 250 |

The following delay mixtures were utilized:

| Material | Mass charged, g |
|---|---|
| Aqueous 4.0 % t-BHP | 124 |
| Aqueous 8% sodium formaldehyde sulfoxylates | 134 |
| Aqueous solution containing 52.5 g Rhodacal DS-10 and 297.5 g water | 264 |
| Monomer Solution comprising 94.76 wt% vinyl acetate, 4.71 wt% acrylic acid, and 0.529 wt% dodecylmercaptan | 540 |
| Ethylene | 400 psig for 3 hours and 1400 psig for 2.5 hours |

Agitation at 100 rpm was begun with a nitrogen purge. Agitation was then increased to 1000 rpm and the reactor heated to 60 °C. After pressurizing the reactor with ethylene to 400 psig (2859 psig), 10 g of 8% aqueous sodium formaldehyde sulfoxylate was added to the reactor. Delay feeds of tert-butylhydrogen peroxide (4%) at 0.4 g/min. and 8% sodium formaldehyde sulfoxylate at 0.4 g/min were begun. At initiation, the surfactant delay was begun at 0.75 g/min and the monomer solution delay was begun at 3.0 g/min. Redox rates were adjusted during the reaction period to maintain reasonable reaction rates. Ethylene pressure of 400 psig was maintained for 3 hours. At the 3 hour mark, the monomer solution delay was turned off and the ethylene pressure was increased to 1400 psig (9,754 kPa). At the 5.5 hour mark, the ethylene makeup valve was closed. The surfactant delay and redox delays were stopped at the 6 hour mark. The reaction was then cooled to 35 °C, transferred to a degasser to remove unreacted ethylene, and 2 g of Rhodaline 675 defoamer was added. The following properties of the resulting emulsion polymer were measured:

| Polymer Composition (by solids calculation) | 24% Ethylene 72.5% Vinyl acetate 3.5% Acrylic Acid |
|---|---|
| T_{g} Onset (°C) | +10.7 |
| Viscosity (60/12 rpm) (cps) | 530/1160 |
| 100/325 mesh grit (ppm) | < 300 / <193 |
| % solids | 40.2 |
| pH | 4.2 |
| Tm (°C) / H_{f} (J/g) | 95.3/24.1 |

It also should be noted that the emulsion polymers disclosed herein and exemplified by examples 1 through 9 can be dried to obtain powders and the powders employed to produce the cosmetic compositions. Techniques for drying the emulsions are those that are common in the industry such as spray drying, and those skilled in the art can use the appropriate techniques, procedures, and equipment to obtain representative powders. These powders can then be used in cosmetic and personal care formulations, as well as other types of materials not discussed in this disclosure, such as particles in plastisols, modifiers for thermoplastic and thermoset composites, etc.

Examples 10 through 18 are general examples illustrating how the disclosed vinyl acetate-ethylene polymers having crystalline ethylene segments may be used in various cosmetic and personal care formulas.

### Example 10

### MASCARA

A mascara containing pigment of the type disclosed in US 6,503,495, and incorporated by reference, is prepared using the following formulation. The polymer of Example 1 is substituted for the carnauba and synthetic waxes, as well as the acrylate and ammonium acrylate copolymers. The use of the polymer from Example 1 in this formulation imparts excellent durability and gloss, longer wear life, easy removability, non-smudging properties and other beauty benefits to the mascara.

| Ingredient | Weight |
|---|---|
| Deionized Water | 40.18 |
| Glycerol Monostearate | 7.25 |
| Black Iron Oxide | 7.25 |
| Quatemium-18 Hectorite | 3.75 |
| Propylene Carbonate | 1.25 |
| Stearic Acid | 2.75 |
| Oleic Acid | 1.00 |
| Triethanolamine | 1.75 |
| Xanthan Gum | 0.10 |
| Trisodium EDTA | 0.10 |
| Polyvinyl Alcohol | 1.50 |
| Polymer from Example 1 | 32.21 |
| Simethicone | 0.20 |
| Lecithin | 1.25 |
| Ethyl Alcohol | 1.00 |
| Benzyl Alcohol | 0.65 |
| Phenoxyethanol | 0.28 |
| Propylparaben | 0.10 |
| Methylparaben | 0.20 |
| Ethylparaben | 0.20 |
| Panthenol | 0.28 |

### Example 11

### EYE SHADOW

An eye shadow is prepared of the type disclosed in US 6,524,597 and incorporated by reference, using the formulation described below. The beeswax and poly(N-acylalkyleneimine)-modified silicone is replaced by the polymer from Example 2. This substitution produces an eye shadow with excellent consistency and little to no cracking without caking, convenient and soft application, and a smooth and moisturizing feel on the skin.

| Ingredient | Weight |
|---|---|
| Fluorine-treated mica (average particle size of 15 microns) | 15.0 |
| Fluorine-treated talc (average particle size of 6 microns) | 15.91 |
| Fluorine-treated nylon powder (average particle size of 5 microns | 5.0 |
| Quatemium-18 Hectorite | 3.75 |
| Zinc stearate | 5.0 |
| Paraben | 0.1 |
| Red color No. 202 | 0.01 |
| Yellow color No. 401 | 0.2 |
| Blue color No. 404 | 0.4 |
| Black iron oxide | 0.03 |
| Titanium oxide | 0.05 |
| Fluorine-treated mica titanium | 40.0 |
| Squalane | 3.0 |
| 2-Ethylhexyl palmitate | 3.0 |
| Perfluoropolyether | 2.0 |
| Polymer from Example 2 | 13.3 |

### Example 12

### LIP GLOSS

A lip gloss is prepared of the type described in US 4,935,228 and incorporated by reference. The polymer from Example 3 replaces the vinyl acetate-ethylene (MW 2000-2500) in the formulation, and imparts the lip gloss with the following properties: good flow during application, high lip shine/gloss, good wear-retention and improved stability.

| Ingredient | Weight |
|---|---|
| Light Mineral Oil | 42.83 |
| Polyethylene (M.W. 1000-1500) | 5.84 |
| Polymer from Example 3 | 5.82 |
| Polybutene | 42.84 |
| Timiron Super Gold | 2.63 |
| D&C Red #6 Lantrol Disp. 15% | 0.02 |

### Example 13:

### HAIR STYLING TONIC

A hair styling formulation is prepared using a formulation of the type disclosed in US 5,104,642 and incorporated by reference, wherein the polyvinyl acetate is replaced with the polymer of Example 4. The hair styling tonic prepared using the polymer from Example 4 offers good manageability, the look and feel of clean hair between washings, a soft hair feel, low tack/stick and excellent retention of the styling effect.

| Ingredient | Weight |
|---|---|
| Polyvinylpyrrolidone | 0.25 |
| Polymer from Example 4 | 3.75 |
| Benzyl Alcohol | 3.00 |
| Polysorbate 80 | 0.20 |
| Perfume | 0.10 |
| Hydroxypropylmethyl cellulose | 0.40 |
| Preservative | 0.30 |
| Water | q.s. to 100 |

### Example 14

### HAIR STYLING GEL

A hair styling gel formulation is prepared by U.S. 5,104,642 and incorporated by reference. The polymer from example 5 replaces poly(t-butyl acrylate). It offers good hold and manageability, a soft non-sticky feel in hair, the look and feel of clean hair between hair washings, and relief from static electricity.

| Ingredient | Weight |
|---|---|
| Polymer from Example 5 | 1.50 |
| Ethyl n-butyrate | 2.50 |
| Carbomer 941 | 0.30 |
| Aminomethyl Propanol | 0.10 |
| Kathon CG | 0.04 |
| Water | q.s. to 100 |

| | |
|---|---|
| Carbomer 941 - available from Noveon. | |

### Example 15:

### HAIRSPRAY

A hair formulation is prepared in accordance with US 5,104,642 and incorporated by reference. In the aqueous based formulation below, the polymer of Example 6 replaces polyvinylpyrrolidone/polyvinyl acetate. The formulation improvements are good hold and manageability, a soft non-sticky feel in hair, the look and feel of clean hair between hair washings, and relief from static electricity.

| Ingredient | Weight |
|---|---|
| Polymer from example 6 | 4.0 |
| Isopropanol | 5.0 |
| Carbopol 1342 | 0.2 |
| Triethanolamine | 0.1 |
| Glydant | 0.4 |
| Water | q.s. to 100 |

| | |
|---|---|
| Carbopol 1342 - available from Noveon. | |
| Glydant - available from Glyco Chem. Co. | |

### Example 16:

### LIP COLOR

A lip color is prepared in accordance with US 5,318,775 and incorporated by reference. The following is a lip color composition where the polymer of Example 2 is substituted for vinyl acetate-ethylene and lanolin wax. This improves the ease of application, wettability, smooth feel, longevity of wear and other beauty benefits of the Lip Color.

| Ingredient | Weight |
|---|---|
| Polymer from Example 2 | 19.47 |
| Silicone Fluid | 15.00 |
| Lanolin Oil | 14.42 |
| Lan. Oil/Stearalkonium hec. | 14.42 |
| Polybutene | 10.81 |
| Methylparaben | 0.14 |
| Butylparaben | 0.14 |
| Propylparaben | 0.14 |
| BHA | 0.04 |
| Lip Gloss Flavor | 0.14 |
| Red #7/Caster Oil blend | 0.41 |
| Yellow iron oxide/Castor Oil blend | 0.83 |
| Titanium dioxide /Castor Oil blend | 3.21 |
| Blue #1/Castor Oil blend | 0.05 |
| Red #6/Castor Oil blend | 0.44 |
| Talc | 22.46 |
| Titanium dioxide pigment | 2.51 |
| Red #7/Talc | 0.51 |
| Red iron oxide Dry Color Mix | 1.82 |
| Yellow iron oxide | 0.18 |
| Ultramarine Blue | 0.45 |

### Example 17:

### FRAGRANCE EMITTING COMPOSITION

A fragrance emitting composition is prepared in accordance with US 4,405,509 and incorporated by reference. In the following composition, the polymer of Example 3 is substituted for paraffin wax, micro wax white 170/180 and vinyl acetate-ethylene. The composition has excellent retention of fragrance during shipping, storage and after application.

| Ingredient | Weight |
|---|---|
| Butyl Rubber/Paraffin Wax 140 (30/70) | 4.50 |
| Polymer from Example 3 | 162 |
| Petrolatum (Witco) | 5.50 |
| Titanium Dioxide | 2.00 |
| Dyes/Pigments | 0.65 |
| Octylphenol-Benzotriazole | 0.25 |
| Octoxybenzophenone | 0.25 |
| Fragrance | 10.00 |

### Example 18

### SUNSCREEN

A sunscreen formulation containing a sunscreen agent is prepared in accordance with US 5,041,281 and incorporated by reference, where the polymer from Example 4 replaces an vinyl acetate-ethylene copolymer. The sunscreen shows good water resistance, substantivity (i.e., retention on the skin), stability, and rub-resistance.

| Ingredient | Weight |
|---|---|
| Water | 64.35 |
| Padimate O | 6.00 |
| Oxybenzone | 2.00 |
| Polymer from Example 4 | 15.00 |
| Eicosene PVP copolymer | 3.00 |
| lsostearic Acid | 5.00 |
| Ammonium Hydroxide | 0.40 |
| Glyceryl Stearate | 2.25 |
| Cetyl Alcohol | 4.50 |
| Isopropyl Myristate | 5.00 |
| Lanolin | 0.10 |
| Allantoin | 0.05 |
| Carbomer 941 | 0.15 |
| Xanthan Gum | 0.20 |
| Fragrance | 0.50 |
| Preservative | 0.50 |

In summary, the vinyl acetate-ethylene copolymer containing crystalline ethylene segments display some unique and interesting properties which are attractive in cosmetic applications. Other potential applications include oil controlling make-up, skin mask, color protection/hair dye applications, hair fixatives, waxing products, etc. They have been noted to accept rheological additives and require a much lower concentration of these additives to hit target rheological behavior. Films from vinyl acetate-ethylene copolymer emulsions, containing crystalline ethylene segments have unique soft "feel", low surface energy, and are hydrophobic, similar to properties imparted by waxes commonly used in cosmetics. However; vinyl acetate-ethylene copolymer emulsions, containing crystalline ethylene segments have polymer type properties as well - it will form a film with mechanical integrity (i.e., adhesion, cohesion, wear resistance) and provide a barrier.

## Claims

1. In a cosmetic composition comprised of a cosmetically acceptable carrier and a polymer, the improvement which comprises:
a vinyl acetate-ethylene polymer formed by aqueous emulsion polymerization having crystalline ethylene segments, said vinyl acetate-ethylene polymer having a crystalline melting point (Tₘ) ranging from 35 to 110 °C measured at a heat rate of 20 °C/minute and,
a tensile storage modulus of at least 1 × 10⁵ dynes/cm² at 115 °C measured at 6.28 rad/sec.

2. The cosmetic composition of claim 1 wherein the polymer is comprised of from 15 to 90% by weight of polymerized units of vinyl acetate and from about 10 to 85% by weight of polymerized units of ethylene based upon the total weight of the polymer.

3. The cosmetic composition of claim 1 wherein the polymer is comprised of from 25 to 80% by weight of polymerized units of vinyl acetate and from about 20 to 75% by weight of polymerized units of ethylene based upon the total weight of the polymer.

4. The cosmetic composition of claim 3 wherein the glass transition temperature is from +25 °C to about -35 °C as measured at a heat rate of 20 °C per minute.

5. The cosmetic composition of claim 1 wherein the polymer is comprised of from 35 to 75% by weight of polymerized units of vinyl acetate and from about 25 to 65% by weight of polymerized units of ethylene based upon the total weight of the polymer.

6. The cosmetic composition of claim 4 wherein polymerized carboxylic acid units are present in said polymer in an amount from about 0.2 to about 10% by weight of said polymer.

7. The cosmetic composition of claim 5 wherein said polymer has a tensile storage modulus of at least 2 × 10⁵ dynes/cm² at 115 °C and measured at 6.28 rad/sec.

8. The cosmetic composition of claim 6 wherein the polymer is comprised of polymerized units of ethylene, vinyl acetate, and acrylic acid or N-methylolacrylamide.

9. The cosmetic composition of claim 1 wherein the polymer is also comprised of polymerized units of a cationic monomer.

10. The cosmetic composition of claim 1 wherein the crystalline heat of fusion of said polymer is from about 5 to 100 joules per gram as measured at a heat rate of 20 °C per minute.

11. The cosmetic composition of claim 1 wherein the crystalline thermal melting point ranges from 50 to 90 °C as measured at a heat rate of 20 °C per minute.

12. The cosmetic composition of claim 1 wherein the crystalline heat of fusion ranges from preferably 15 to 70 joules per gram as measured at a heat rate of 20 °C per minute.

13. The cosmetic composition of claim 1 wherein said composition is a mascara composition and said cosmetically acceptable carrier contains a pigment.

14. The cosmetic composition of claim 1 wherein said cosmetic composition comprises a fatty oil and water.

15. The cosmetic composition of claim 1 wherein the composition is a sunscreen and contains a sunscreen agent.

16. The cosmetic composition of claim 1 wherein the cosmetically acceptable carrier is designed as a hair spray formulation.
